# EUROPEAN PATENT SPECIFICATION

(11) **EP 1 980 239 B1**
(45) Date of publication and mention of the grant of the patent: **11.01.2012**
(21) Application number: 08251148.6
(22) Date of filing: 28.03.2008
(51) Int. Cl.: A61K 8/97, A61Q 1/06, A61Q 1/08, A61Q 1/10, A61Q 1/12, A61Q 1/04

(54) **Cheek tint cosmetic composition comprising natural pigments**
Kosmetische Rouge Zusammensetzungen enthältend natürlichen Pigmenten
Fard à joues comprenant des pigments naturels

(30) Priority: 29.03.2007 US 693615; 29.03.2007 US 693634
(43) Date of publication of application: 15.10.2008
(73) Proprietor: Velveteen Bunni, LLC, Oakland, CA 94607 (US)
(72) Inventor: Kostick, Richard Hughes, Oakland, CA 94607 (US); Wang, Susie Pei Hwa, Oakland, CA 94607 (US); Wang, James Pei Fung, Oakland, CA 94607 (US)
(74) Representative: Gates, Marie Christina Esther

(56) References cited:
- WO-A-02/47634
- WO-A-2006/117465
- JP-A- 2000 302 663
- JP-A- 2007 046 015
- US-A- 5 997 889
- US-A1- 2002 187 115
- US-A1- 2004 170 583
- US-A1- 2006 280 762

## Description

### FIELD OF INVENTION

Cosmetic and dermatological formulations.

### BACKGROUND OF INVENTION

The use of cosmetics is widespread in modem society. Cosmetics typically are intended to provide an attractive appearance through the use of color, e.g., by highlighting certain features of the face and/or accentuating natural colors. Colored cosmetics are used, for example, to accentuate lines of separation (eye liners and lip liners), to provide sensuous color to portions of the skin (lipsticks and glosses) and to provide a "healthy glow" to the cheeks (blushes and rouges). Cosmetics may also be used to hide imperfections of the skin and to protect the skin, e.g., by blocking the skin from harmful ultraviolet light.

A variety of coloring agents are typically used to color cosmetics, including inorganic and organic synthetic dyes or pigments. Many cosmetic manufacturers use artificial or man-made pigments approved by the Food and Drug Administration designated as an FD&C "color" followed by a number. Examples of such pigments include FD&C Blue No. 1, FD&C Blue No. 2, FD&C Green No. 3, FD&C Red No. 3, FD&C Red No. 40, FD&C Yellow No. 5, FD&C Yellow No. 6, D&C Blue No. 4, D&C Blue No. 9, D&C Green No. 5, D&C Green No. 6, D&C Green No. 8, D&C Orange No. 4, D&C Orange No. 5, D&C Orange No. 10, D&C Orange No. 11, D&C Orange No. 17, FD&C Red No. 4, D&C Red No. 6, D&C Red No. 7, D&C Red No. 8, D&C Red No. 9, D&C Red No. 17, D&C Red No. 19, D&C Red No. 21, D&C Red No. 22, D&C Red No. 27, D&C Red No. 28, D&C Red No. 30, D&C Red No. 31, D&C Red No. 34, D&C Red No. 39, FD&C Red No. 40, D&C Violet No. 2, D&C Yellow No. 7, Ext. D&C Yellow No. 7, D&C Yellow No. 8, D&C Yellow No. 11, D&C Brown No. 1, Ext. D&C Violet No. 2, D&C Blue No. 6 and D&C Yellow No. 10.

Generally, to be useful as cosmetic colorants, soluble dyes must be converted into insoluble forms. Any soluble dye that is in solution can be referred to as a "solvated dye". A solvated dye composed of a water-soluble dye in an aqueous solution can be referred to as an "aqueous dye". There are a variety of methods currently used in the cosmetic industry to insolubilize soluble dyes. A common method used to insolubilize water-soluble dyes is called "laking". "Lake" colorants are metallic complexes of organic coloring matter obtained by precipitating an organic dyestuff onto an inorganic substrate. *See generally* U.S. Pat. No. 3,873,687. Water-soluble dyes have also been insolubilized by salifying the dyes with copolymers. *See generally* U.S. Pat. No. 4438,140.

Although FD&C colors are primarily used to formulate a cosmetic product, another class of pigments that are conventionally used are minerals, which are materials extracted from the earth, including, for example, iron oxides. Also used to formulate cosmetics are animal byproducts, such as blood and carmine.

### SUMMARY OF INVENTION

Pigments from plant products can be combined with a cosmetic carrier formulation to create a cosmetic and/or dermatological product for transferring color to the skin of the person. A plant product extract or reformulation, such as a concentrate, flake or powder of the plant, can be combined with a cosmetic carrier formulation to create a cosmetic and/or dermatological product for transferring color to the skin of the person. Fruits, vegetables, seeds and legumes can be used to extract natural pigment or reformulate a plant product into a concentrate, flake or powder. Resultant cosmetic products can include lipstick, lip gloss, lip stain, lip liner, blush, face tint, cheek stain, cheek gel, cheek butter, eye shadow, eyebrow powder, eyeliner, mascara, foundation, sheer foundation, bronzer, facial illuminator, facial highlighter, face powder, lotion and tinted moisturizer.

The present invention provides for a cheek tint as recited in the appended claims.

### DETAILED DESCRIPTION

According to embodiments of the invention, pigments extracted from cherry and cranberry fruits, can be used as a colorant in cosmetic and dermatological products. The colorant is suitable for transfer to the skin when the cosmetic and/or dermatological product is applied thereon. In this manner, the pigment or reformulant is a "staining" coloranl used to highlight and accentuate natural colors of the face Such natural pigments can replace artificial, man-made pigments commonly used in cosmetic and dermatological products.

Generally, fruits and vegetables are composed of a variety of compounds, including, water, carbohydrates, fats, lipids, proteins, amino acids, vitamins, minerals (e.g., phosphorous, potassium, calcium, magnesium and iron), organic acids, pectins, pectic enzymes and odorous compounds. Fruits and vegetables also contain pigments including, but not limited to, chlorophylls, anthoxanthins, betacyanins, carotenoids such as xanthaphylls and carotenes, and flavonoids such as flavones and anthocyanidins (e.g., cyanidin, pelarganodin, delphinidin, malvidin, paenidin).

Anthocyanins, which are anthocyanidins with a sugar group, are water-soluble vacuolar flavonoid pigments that appear red to blue, according to pH. They are synthesized exclusively by organisms of the plant kingdom, and have been observed to occur in all tissues of higher plants; providing color in leaves stems, roots, flowers, and fruits. Anthocyanin pigment is present in fruits such as, but not limited to, strawberries, pomegranates, cranberries, cherries and berries, and is responsible for their red color. Anthocyanins are a sub-category of flavonoids, and flavonoids are a sub-category of polyphenols. Lycopene is a bright red carotenoid pigment, a phytochemical found in tomatoes and other red fruit. Lycopene is a sub-category of tetraterpenes, and Ictraterpenes are a sub-category of terpenes. Chlorophyll pigment is present in vegetables such as, but not limited to, green vegetables including beet greens, bok choy, collards, dandelion greens, kale, mustard greens and blue-green algae and is responsible for their green color. Carotenoid pigment, a carotene, is present in, for example, carrots and is responsible for their bright orange color. Anthoxanthin pigment is present in, for example, alfalfa flowers and red cabbage and is naturally a white color.

The skin or pulp of fruit or vegetables can be processed to extract [pigments such as anthocyanins and lycopenes. The resulting pigment extract can be in liquid or solid form. These various pigments in the form of extractions can be combined to produce numerous colors and used to formulate cosmetic and/or dermatological products according to embodiments of the invention. These plant products can be subjected to a process to isolate the pigments therefrom to be used to formulate a cosmetic or dermatological product according to some embodiments of the invention.

### Example 1

In one example, a plant sample, such as the seed, skin or pulp of fruit can be subjected to a "quick freeze" in liquid nitrogen and then subsequently freeze-dried. The sample can thereafter be subjected to at least 3 washes with hexane. The hexane washes are used to remove lipid from the plant sample (if the sample contains lipids). The hexane should preferably be optima grade hexane (available from Fischer Scientific, Waltham, Massachusetts) or equivalent. Polyphenols in the sample can then be extracted using a solvent system such as methanol/water (75:25 v/v). The methanol should preferably be high-performance liquid chromatography (HPLC) grade methanol (available from Sigma-Aldrich, St. Louis, Missouri) and the water should preferably be purified to about 18.2 megaohms per centimeter (MΩ/cm). In some embodiments, the extraction step can be performed multiple times. The extract can then be filtered through a 0.45 micron polytetrafluoroethylene (PTFE) membrane, or other size depending on the sample size.

### Example 2

In another example, a plant sample such as the seed, skin or pulp of fruit can be subjected to a "quick freeze" in liquid nitrogen and then subsequently freeze-dried-The sample can be ground down using a mortar and pestle in optima grade hexane. The sample can then be washed with hexane and centrifuged for about 10 minutes at a speed of about 4000 to 5000 rotations per minute. This removes lipids from the sample (if the sample contains lipids). This can be done for at least 3 cycles, letting the particulates settle between each wash/centrifugation. Thereafter, the remaining Precipitate is extracted using a solvent system such as methanol/water (75.25 v/v) for about 24 hours at approximately 5 degrees Celsius.

In other embodiments, fruit or vegetables can be reformulated to obtain a powder, flake or concentrate (collectively, plant product reformulation) of the fruit or vegetable, which also can include the pigments described previously. This plant product reformulation, or reformulant, can also be used to formulate cosmetic and/or dermatological products according to embodiments of the invention In the context of this Application, "reformulate" means to alter a plant product from its natural state to an altered state such as a concentrate, flake or powder.

In one example, to reformulate a vegetable into a powder, the following alternative processes can be used; (a) dry vegetable down to a final water content below 4%, followed by grinding, followed by sieving; or (b) boil vegetable, followed by sieving into a puree, followed by drying on a heated surface (preferably under vacuum) or spraying in hot air. The drying processes can encompass one of the following: (i) dryers with plates under vacuum are equipped with plates heated with hot water; stainless steel plates containing purée to be dried are placed on them; process conditions are at low residual pressure (about 10 to 20 mm Hg) and a product temperature of between 50°C to 70°C; (ii) drum dryers having one or two drums heated with hot water or steam as heating elements; feeding is continuous between the two drums which can rotate in reverse direction (about 2-6 rotations per minute) and the distance of which is adjustable and determines the thickness of layer to be dried; the product can be dried and removed by mechanical means during rotation; or (iii) drying installations by spraying in hot air; the product is introduced in equipment and sprayed by a special device in hot air; drying is instantaneous (1/50 s) and therefore can be carried out in a range of between 130°C to 150°C.

In another example, to reformulate a fruit such as a tomato into a concentrate, the following process can be used: pre-wash, wash and sort tomatoes, hollowed by tomato crushing and seed separation with a centrifugal separator. The resulting tomato pulp is pre-healed at 55°C to 60°C and then passed to an equipment group for sieving: pulper, refiner and super-refiner with sieves of 1.5 mm, 0.8 mm and 0.4 to 0.5 mm, respectively. The resulting tomato juice is concentrated by vacuum evaporation. For example, the tomato juice can be subjected to a 3-step evaporation method: (i) pasteurize juice at between 85°C to 90°C for 15 minutes at 330 mm Hg; (ii) heat resulting concentrate at between 42°C to 46°C at between 680 mm Hg to 700 mm Hg; and heat concentrate again at between 42°C to 46°C at between 680 mm Hg to 700 mm Hg. Force resulting concentrate through tubular pasteurizer at between 90°C and 92°C.

Alternatively, freeze drying or dehydration of a fruity vegetable, fruit skin or vegetable skin can result in fruit or vegetable, powder or flakes that can be used to formulate cosmetic and/or dermatological products according to embodiments of the invention. "Freeze drying" is the process of freezing a material and then reducing the surrounding pressure and adding enough heat to allow the frozen water in the material to sublime directly from the solid phase to gas. "Dehydration" is the process of removing water from an object. Dehydration of fruits and methods can be performed by known methods, In some embodiments, the dehydrated (or freeze dried) plant product can be micronized to create the powder. "Micronized" means to reduce to particles that are only a few microns in diameter. In some embodiments, the concentrate, powder or flakes can be the direct source of color transfer from the cosmetic and/or dermatological product, eliminating the need for color additives such as FD&C colors and/or Lake minerals. However, those skilled in the an will appreciate that the use of FD&C colors and/or Lake minerals in embodiments of the cosmetic and/or dermatological formulations of the invention may also be included.

The pigment extracts and/or plant product reformulations can be combined with, for example, a cosmetic carrier. A cosmetic carrier is a formulation that can be safely applied to the skin of a person and is principally used for beautifying effects and ease of application. The cosmetic carrier may be composed of a wide variety of ingredients that are conventionally used in cosmetics, e.g., waxes, mineral oils, fatty alcohols, glycerine, and sunscreens. In one embodiment, the cosmetic carrier may be a combination of shea butter, cocoa butter, grapeseed oil, honey beeswax, vitamin C, vitamin E and a natural flavor. When the pigment extract or plant product reformulation (e.g., powder, flakes, concentrate) are combined with a cosmetic carrier, the resulting formulation is used to make a blush, face tint, cheek stain, cheek gel, cheek butter.

The cheek tint of the invention (e.g., shimmery strawberry) can include 32.0% cocoa butter, 25.0% shea butter, 13.0% grapeseed oil, 10.0% honey beeswax, 5.0% vitamin E, 2.8% cranberry fruit pigment, 2.0% cherry fruit pigment, 2.0% vitamin C and 1.5% natural strawberry flavor.

When applied, the fruit pigment formulated within the cosmetic or dermatological product can stain the skin immediately on application. In some embodiments, there is a time interval between the time the cosmetic and/or dermatological product is applied to the skin and the time that a pigment in the product stains the skin. A representative time interval from application to staining can be up to about 5 minutes, and, in one embodiment, 30 seconds to one minute.. In any case, the stain is temporary, not permanent.

## Claims

1. A cheek tint comprising:
2.8% cranberry fruit pigment;
2.0% cherry fruit pigment; and
a cosmetic carrier, wherein the cosmetic carrier includes 32.0% cocoa butter, 25.0% shea butter, 13.0% grapeseed oil, 10.0% honey beeswax, 5.0% vitamin E, 2.0% vitamin C, and 1,5% natural strawberry flavor.

## Patentansprüche

1. Rouge, umfassend:
2,8% Moosbeerenfruchtpigment;
2,0% Kirschfruchtpigment; und
einen kosmetischen Träger, wobei der kosmetische Träger 32,0% Kakaobutter, 25,0% Sheabutter, 13,0% Traubenkernöl, 10,0% Bienenwachs, 5,0% Vitamin E, 2,0% Vitamin C und 1,5% natürliches Erdbeeraroma umfasst.

## Revendications

1. Fard à joue comprenant :
2,8 % de pigment de canneberge ;
2,0 % de pigment de cerise ; et
un véhicule cosmétique, le véhicule cosmétique comprenant 32,0 % de beurre de cacao, 25,0 % de beurre de karité, 13,0 % d'huile de pépins de raisin, 10,0 % de cire d'abeilles, 5,0 % de vitamine E, 2,0 % de vitamine C et 1,5 % d'arôme naturel de fraise.
